# EUROPÄISCHE PATENTSCHRIFT

(11) **EP 0 759 426 B1**
(45) Veröffentlichungstag und Bekanntmachung des Hinweises auf die Patenterteilung: **28.11.2001**
(21) Anmeldenummer: 96112609.1
(22) Anmeldetag: 05.08.1996
(51) Int. Cl.: C07C 209/58, C07B 53/00, C07C 231/16, C12P 13/02

(54) **Verfahren zur Herstellung von optisch aktiven 1-Aryl-alkylaminen**
Process for the preparation of optically active 1-aryl-alkylamines
Procédé pour la préparation d'aryl-1-alkylamines optiquement actives

(30) Priorität: 17.08.1995 DE 19530204
(43) Veröffentlichungstag der Anmeldung: 26.02.1997
(73) Patentinhaber: BAYER AG, 51368 Leverkusen (DE)
(72) Erfinder: Stelzer, Uwe, Dr., 51399 Burscheid (DE)

(56) Entgegenhaltungen:
- WO-A-95/07359
- WO-A-95/08636
- DE-A- 2 357 749
- PATENT ABSTRACTS OF JAPAN vol. 096, no. 003, 29. März 1996 & JP 07 303496 A (ASAHI CHEM IND CO LTD), 21. November 1995
- PATENT ABSTRACTS OF JAPAN vol. 095, no. 004, 31. Mai 1995 & JP 07 010813 A (NISSAN CHEM IND LTD), 13. Januar 1995
- MARIAN C. DE ZOETE ET AL.: "Ester ammoniolysis: a new enzymatic reaction" JOURNAL OF THE CHEMICAL SOCIETY, CHEMICAL COMMUNICATIONS., Bd. 24, 1993, Seiten 1831-1832, XP002058441 LETCHWORTH GB

## Beschreibung

Die vorliegende Erfindung betrifft ein neues Verfahren zur Herstellung von (R)-1-Aryl-alkylaminen, die als Zwischenprodukte zur Synthese von Wirkstoffen mit fungiziden Eigenschaften verwendet werden können.

Es ist bereits bekannt, daß sich optisch aktive Amine herstellen lassen, indem man Racemate von Aminen mit 2-(4-Hydroxyphenoxy)-propionsäure umsetzt, aus dem entstehenden Diastereomeren-Gemisch das gewünschte Produkt abtrennt und daraus durch Behandlung mit Base das optisch aktive Amin freisetzt (vgl. JP-A 95-010 813). Ungünstig an diesem Verfahren ist, daß die Ausbeuten an den gewünschten Substanzen nicht immer befriedigend sind und vor allem die optische Reinheit der Reaktionsprodukte zu wünschen übrig läßt.

Weiterhin ist schon bekannt, daß man (R)-1-(4-Chlor-phenyl)-ethylamin erhält, wenn man racemisches 1-(4-Chlor-phenyl)-ethylamin in Gegenwart von Ethanol mit (S)-(-)-N-Phenylcarbamat-milchsäure umsetzt, den dabei entstehenden Kristallbrei absaugt und mit wäßriger Natronlauge in Gegenwart von Methylenchlorid behandelt (vgl. EP-A 0 341 475). Die Wirtschaftlichkeit dieses Verfahrens wird aber dadurch beeinträchtigt, daß ein beachtlicher Anteil an (S)-(-)-N-Phenylcarbamat-milchsäure in der nach dem Absaugen des Kristallbreis anfallenden Mutterlauge vorhanden ist und daraus nur in aufwendiger Weise isoliert werden kann.

Wie ferner in der DE-A 4 332 738 offenbart wird, lassen sich optisch aktive primäre und sekundäre Amine herstellen, indem man zunächst racemisches Amin mit bestimmten Estern in Gegenwart einer Hydrolase enantioselektiv acyliert, dann das Gemisch aus optisch aktivem Amin und optisch aktivem acyliertem Amin trennt und dadurch ein Enantiomer des Amins erhält und gegebenenfalls aus dem optisch aktiven, acylierten Amin durch Amidspaltung das andere Enantiomer erhält. Beim Arbeiten nach diesem Verfahren entstehen aber jeweils nur die (S)-Amine. Um auch die entsprechenden (R)-Amine zu gewinnen, ist eine aufwendige Abtrennung der acylierten Komponente und deren Spaltung erforderlich.

Aus. J. Chem. Soc. Chem. Commun. 1993, 1831-1832 ist zu entnehmen, daß sich Racemate des 2-(4-Isobutyl-phenyl)-propionsäure-(2-chlorethyl)-esters mit Hilfe von Ammoniak in Gegenwart bestimmter Enzyme, wie Candida antarctica Lipase SP 435, enantioselektiv in das (R)-2-(4-Isobutyl-phenyl)-propionsäure-amid überführen lassen. Dieses Verfahren ist aber mit dem Nachteil behaftet, daß neben dem gewünschten (R)-Amid auch ein erheblicher Anteil an freier (R)-Säure entsteht.

Darüber hinaus ist bekannt, daß optisch aktive Amine durch enantioselektive Hydrierung von C = N-Bindungen in Gegenwart von chiralen Übergangsmetall-Komplexen zugänglich sind (vgl. Angew. Chem. 105, 245 (1993)). Neben einer aufwendigen Herstellung und Rückführung des Katalysators weist dieses Verfahren den Nachteil auf, daß die optischen Ausbeuten in manchen Fällen relativ niedrig sind.

Schließlich geht aus der Literatur hervor, daß optisch aktive Amine bei der asymmetrischen Reduktion von Oximen mit Lithium-aluminium-hydrid, chiralen Lithium-aluminium-hydrid-Komplexen oder chiralen Boran-Komplexen in guten optischen Ausbeuten anfallen (vgl. Chem. Lett. (1986), 1133 und J. Chem. Perkin Trans. I (1985), 2039). Für eine Durchführung im technischen Maßstab ist diese Methode aber aus Sicherheitsgründen nur bedingt geeignet.

Es wurde nun gefunden, daß man (R)-1-Aryl-alkylamine der Formel 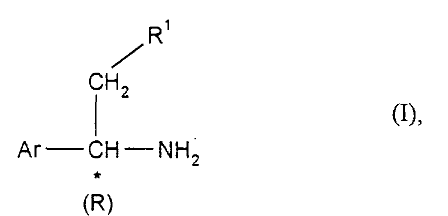 in welcher
- R¹: für Wasserstoff oder Alkyl steht und
- Ar: für gegebenenfalls substituiertes Phenyl der Formel 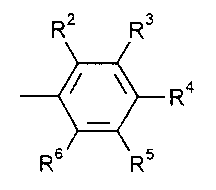 steht, worin
R² und R⁶ für Wasserstoff oder Fluor stehen und
R³, R⁴ und R⁵ unabhängig voneinander für Wasserstoff, Halogen, Alkyl, Halogenalkyl, Alkoxy, Halogenalkoxy, Cyano, Dialkylamino, Nitro, Phenyl, Phenoxy oder Benzyl stehen, oder
- Ar: für gegebenenfalls einfach bis dreifach, gleichartig oder verschieden durch Halogen, Alkyl, Halogenalkyl, Alkoxy und/oder Halogenalkoxy substituiertes Naphthyl steht,
erhält, indem man
a) Racemate von 2-Aryl-alkansäure-ethylestem bzw. -methylestern der Formel 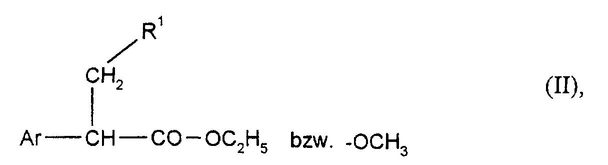 in welcher
   - R¹ und Ar: die oben angegebenen Bedeutungen haben,
   mit Ammoniak in Gegenwart von Lipasen, die zur Spaltung von Estern geeignet sind, in Gegenwart eines Verdünnungsmittels umsetzt,
b) aus dem Reaktionsgemisch die entstandenen (R)-2-Aryl-alkansäure-amide der Formel 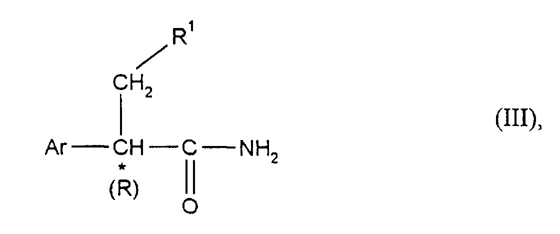
in welcher
- R¹ und Ar: die oben angegebenen Bedeutungen haben,
abtrennt und dann mit Natrium-hypohalogeniten der Formel

NaOHal (IV),

in welcher
- Hal: für Chlor oder Brom steht,
in Gegenwart von Wasser umsetzt.

Unter (R)-1-Aryl-alkylaminen sind diejenigen optisch aktiven Verbindungen der Formel (I) zu verstehen, die am asymmetrisch substituierten Kohlenstoffatom die (R)-Konfiguration aufweisen. In der Formel (I) ist das asymmetrisch substituierte Kohlenstoffatom durch (*) gekennzeichnet. In gleicher Weise sind die Chiralitätszentren auch in anderen Formeln markiert.

Es ist als äußerst überraschend zu bezeichnen, daß sich (R)-1-Aryl-alkylamine nach dem erfindungsgemäßen Verfahren in hoher Ausbeute und sehr guter optischer Reinheit herstellen lassen. Aufgrund des bekannten Standes der Technik konnte nämlich nicht damit gerechnet werden, daß die im Vergleich zu den 2-Chlorethylestern weniger reaktiven Ethylester und Methylester sich als Ausgangsstoffe eignen würden. Unerwartet ist auch, daß eine Hydrolyse der eingesetzten Ester nur in sehr geringem Maße stattfindet.

Das erfindungsgemäße Verfahren weist eine Reihe von Vorteilen auf. So ermöglicht es die Herstellung einer Vielzahl von (R)-1-Aryl-alkylaminen in hoher Ausbeute und sehr guter optischer Reinheit. Günstig ist auch, daß sowohl die Ausgangsstoffe als auch die Reaktionskomponenten in einfacher Weise zugänglich sind und auch in größeren Mengen zur Verfügung stehen. Von besonderem Vorteil ist schließlich, daß sich auch der jeweils nicht umgesetzte (S)-2-Aryl-alkansäureester in einfacher Weise racemisieren und erneut zur Racematspaltung verwenden läßt.

Setzt man racemischen 2-(4-Chlorphenyl)-propionsäure-ethylester mit Lipase aus Candida antarctica in Gegenwart von Ammoniak um und behandelt man das daraus entstandene Amid nach vorheriger Abtrennung mit Natriumhypobromit, so kann der Verlauf des erfindungsgemäßen Verfahrens durch das folgende Formelschema veranschaulicht werden.

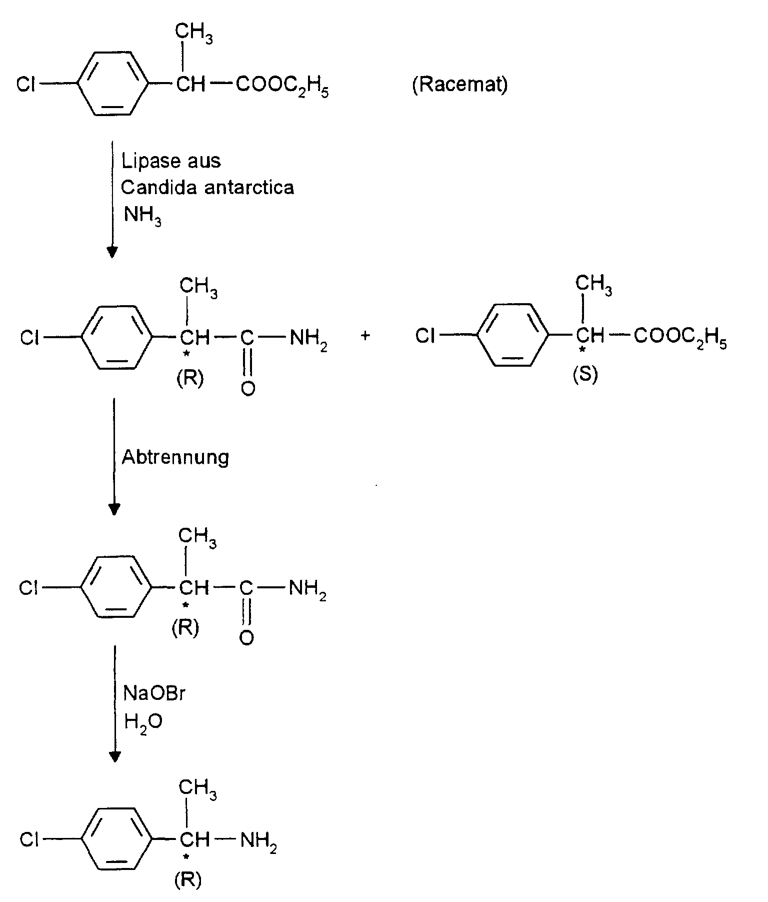

Die bei der Durchführung des erfindungsgemäßen Verfahrens als Ausgangsstoffe benötigten racemischen 2-Aryl-alkansäure-ethylester bzw. -methylester sind durch die Formel (II) allgemein definiert. In dieser Formel steht
- R¹: vorzugsweise für Wasserstoff oder geradkettiges oder verzweigtes Alkyl mit 1 bis 4 Kohlenstoffatomen, und
- Ar: steht für gegebenenfalls substituiertes Phenyl der Formel 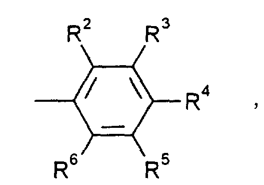 worin
R² und R⁶ vorzugsweise für Wasserstoff oder Fluor stehen, wobei zumindest einer dieser beiden Substituenten für Wassersstoff steht, und
R³, R⁴ und R⁵ unabhängig voneinander vorzugsweise für Wasserstoff, Fluor, Chlor, Brom, geradkettiges oder verzweigtes Alkyl mit 1 bis 4 Kohlenstoffatomen, geradkettiges oder verzweigtes Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Cyano, Dialkylamino mit 1 bis 4 Kohlenstoffatomen in jeder Alkylgruppe, Nitro, Phenyl, Phenoxy oder Benzyl stehen, oder
Ar steht vorzugsweise für gegebenenfalls einfach bis dreifach, gleichartig oder verschieden durch Fluor, Chlor, Brom, geradkettiges oder verzweigtes Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen und/oder Halogenalkoxy mit 1 bis 4 Kohlenstoffatomen und 1 bis 5 gleichen oder verschiedenen Halogenatomen substituiertes Naphthyl, wobei jedoch die ortho-Positionen zu dem Kohlenstoffatom, über das der Naphthyl-Rest gebunden ist, nicht substituiert sind.
Besonders bevorzugt sind Verbindungen der Formel (II), in denen
- R¹: für Wasserstoff, Methyl, Ethyl, n-Propyl, Isopropyl, n-Butyl, iso-Butyl oder sek.-Butyl steht und
- Ar: für gegebenenfalls substituiertes Phenyl der Formel 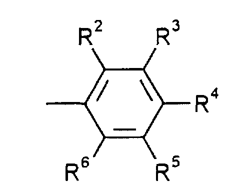 steht, worin
R² und R⁶ für Wasserstoff oder Fluor stehen, wobei zumindest einer dieser beiden Substituenten für Wassersstoff steht, und
R³, R⁴ und R⁵ unabhängig voneinander vorzugsweise für Wasserstoff, Fluor, Chlor, Brom, Methyl, Ethyl, n-Propyl, Isopropyl, n-Butyl, iso-Butyl, sek.-Butyl, tert.-Butyl, Methoxy, Ethoxy, Trichlormethyl, Trifluormethyl, Difluormethyl, Trifluormethoxy, Difluorchlormethoxy, Difluormethoxy, Cyano, Dimethylamino, Diethylamino, Nitro, Phenyl, Phenoxy oder Benzyl stehen, oder
- Ar: für gegebenenfalls einfach bis dreifach, gleichartig oder verschieden durch Fluor, Chlor, Brom, Methyl, Ethyl, n-Propyl, Isopropyl, n-Butyl, iso-Butyl, sek.-Butyl, tert.-Butyl, Methoxy, Ethoxy, Trichlormethyl, Trifluormethyl, Difluormethyl, Trifluormethoxy und/oder Difluormethoxy substituiertes Naphthyl steht, wobei jedoch die ortho-Positionen zu dem Kohlenstoffatom, über das der Naphthyl-Rest gebunden ist, nicht substituiert sind.

Die racemischen 2-Aryl-alkansäure-ethylester bzw. -methylester der Formel (II) sind bekannt oder lassen sich nach bekannten Methoden herstellen.

Als Biokatalysatoren kommen bei der Durchführung der ersten Stufe des erfindungsgemäßen Verfahrens alle diejenigen Lipasen in Betracht, die selektiv zur Spaltung der (R)-Enantiomeren von 2-Aryl-alkansäure-ethylestern der Formel (II) und zur Katalyse der Amidbildung geeignet sind. Als Beispiele seien Lipase aus Candida antarctica, Newlase F, Lipase aus Pseudomonas sp. und Lipase M genannt. Diese als Biokatalysatoren einsetzbaren Lipasen sind bekannt.

Die Lipasen können bei der Durchführung der ersten Stufe des erfindungsgemäßen Verfahrens entweder nativ oder in modifizierter Form, z.B. mikroverkapselt oder an anorganische oder organische Trägermaterialien gebunden eingesetzt werden.

Als Trägermaterialien kommen dabei zum Beispiel Celite, Zeolithe, Polysaccharide, Polyamide und Polystyrolharze in Frage.

Zur Aminolyse dient bei der Durchführung der ersten Stufe des erfindungsgemäßen Verfahrens Ammoniak. Dieser wird vorzugsweise getrocknet entweder gasförmig oder in flüssiger Form eingesetzt.

Als Verdünnungsmittel kommen bei der Durchführung der ersten Stufe des erfindungsgemäßen Verfahrens vorzugsweise tertiäre Alkohole, wie tert.-Butanol und 3-Methyl-3-pentanol, ferner Ether, wie Dioxan, Furan, Dimethylether, Methyl-tert.-butyl-ether und tert.-Amyl-methyl-ether, sowie auch Acetonitril und Isopropanol in Betracht. Besonders bevorzugt verwendbar ist tert.-Butanol.

Die Temperaturen können bei der Durchführung der ersten Stufe des erfindungsgemäßen Verfahrens innerhalb eines bestimmten Bereiches variiert werden. Im allgemeinen arbeitet man bei Temperaturen zwischen -10°C und 80°C, vorzugsweise zwischen 0°C und 70°C.

Bei der Durchführung der ersten Stufe des erfindungsgemäßen Verfahrens arbeitet man entweder unter Atmosphärendruck oder, sofern flüssiger Ammoniak in einem Druckgefäß eingesetzt wird, unter dem sich dabei einstellenden Eigendruck. Es ist aber auch möglich, unter zusätzlich erhöhtem Druck zu arbeiten.

Bei der Durchführung der ersten Stufe des erfindungsgemäßen Verfahrens setzt man auf 1 g an racemischem 2-Aryl-alkansäure-ethylester oder -methylester der Formel (II) im allgemeinen 0,01 g bis 0,5 g, vorzugsweise 0,01 g bis 0,3 g an Lipase ein. Die Menge an Ammoniak kann innerhalb eines größeren Bereiches variiert werden. Auf 1 Mol an racemischem 2-Aryl-alkansäure-ethylester bzw. methylester der Formel (II) setzt man im allgemeinen 0,5 Mol bis 30 Mol, vorzugsweise 0,5 Mol bis 10 Mol an Ammoniak ein. Beim Arbeiten mit gasförmigem Ammoniak geht man im einzelnen so vor, daß man das Reaktionsgemisch zunächst bis zur Sättigung mit Ammoniak versetzt und dann bis zur Beendigung der Umsetzung Ammoniak durch das Reaktionsgefäß leitet. Beim Arbeiten mit flüssigem Ammoniak verfährt man in der Weise, daß man flüssigen Ammoniak bei niedriger Temperatur in einem mit dem Reaktionsgemisch gefüllten Druckgefäß kondensiert, dann das Druckgefäß verschließt und auf die jeweils gewünschte Temperatur erwärmt. Die Aufarbeitung erfolgt nach üblichen Methoden. Im allgemeinen geht man so vor, daß man den Biokatalysator abtrennt, das Filtrat einengt und den verbleibenden Rückstand in einem geeigneten Lösungsmittel-Gemisch aufnimmt, so daß das gewünschte Amid in fester Form ausfällt und abfiltriert werden kann. Aus dem Filtrat kann durch Einengen unter vermindertem Druck der (S)-2-Aryl-alkansäure-ethylester bzw. -methylester gewonnen werden. Dieser läßt sich z.B. durch Behandeln mit Natriumethylat in Ethanol racemisieren und erneut für die Racematspaltung verwenden.

Als Hypohalogenite kommen bei der Durchführung der zweiten Stufe des erfindungsgemäßen Verfahrens Natriumhypochlorit und Natriumhypobromit in Betracht. Im allgemeinen geht man so vor, daß man diese Stoffe frisch herstellt, indem man Chlor oder Brom mit wäßriger Natronlauge umsetzt und die weitere Reaktion unmittelbar anschließt.

Die Reaktionstemperaturen können bei der Durchführung der zweiten Stufe des erfindungsgemäßen Verfahrens innerhalb eines bestimmten Bereiches variiert werden. Im allgemeinen arbeitet man bei Temperaturen zwischen 0°C und 120°C, vorzugsweise zwischen 20°C und 100°C.

Bei der Durchführung der zweiten Stufe des erfindungsgemäßen Verfahrens arbeitet man im allgemeinen unter Atmosphärendruck. Es ist aber auch möglich, unter erhöhtem Druck zu arbeiten.

Bei der Durchführung der zweiten Stufe des erfindungsgemäßen Verfahrens setzt man auf 1 Mol an (R)-2-Aryl-alkansäureamid der Formel (III) im allgemeinen 1,1 bis 10 Mol, vorzugsweise 1,1 bis 5 Mol an Natriumhypohalogenit ein. Die Aufarbeitung erfolgt nach üblichen Methoden. Im allgemeinen verfährt man in der Weise, daß man das Reaktionsgemisch mit einem mit Wasser wenig mischbaren, organischen Lösungsmittel extrahiert, die vereinigten organischen Phasen trocknet und unter vermindertem Druck einengt. Gegebenenfalls kann eine weitere Reinigung der anfallenden Produkte, z.B. durch Chromatographie oder Destillation, vorgenommen werden.

Die nach dem erfindungsgemäßen Verfahren herstellbaren (R)-1-Aryl-alkylamine der Formel (I) sind wertvolle Zwischenprodukte zur Herstellung von Pharmazeutika oder von Wirkstoffen mit insektiziden, fungiziden oder herbiziden Eigenschaften (vgl. EP-A 0 519 211, EP-A 0 453 137, EP-A 0 283 879, EP-A 0 264 217 und EP-A 0 341 475). So erhält man z.B. die fungizid wirksame Verbindung der Formel 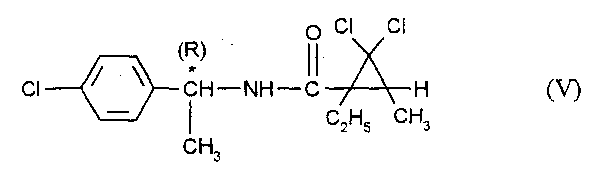 indem man das (R)-1-(4-Chlor-phenyl)-ethylamin der Formel 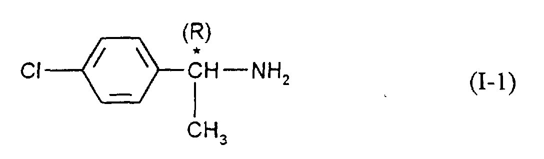 mit 2,2-Dichlor-1-ethyl-3-methyl-1-cyclopropancarbonsäurechlorid der Formel 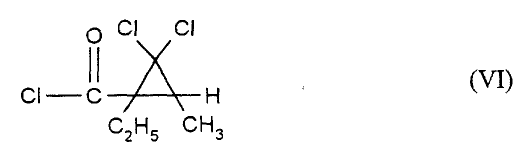 in Gegenwart eines Säurebindemittels und in Gegenwart eines inerten organischen Verdünnungsmittels umsetzt.

Die Durchführung des erfindungsgemäßen Verfahrens wird durch die folgenden Beispiele veranschaulicht.

### Herstellungsbeispiele

### Beispiel 1

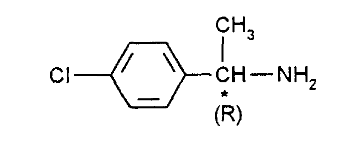

### 1. Stufe:

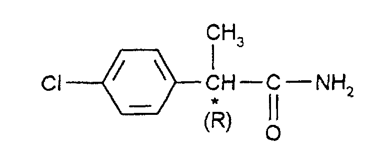

### Variante α:

Ein Gemisch aus 5 g racemischem 2-(4-Chlor-phenyl)-propionsäure-ethylester und 50 ml tert.-Butanol wird bei 50°C unter Rühren mit 1 g Lipase aus Candida antarctica (Novozym 435, Aktivität 7000 PLU/g) versetzt. Danach wird unter Rühren bei 50°C bis zur Sättigung Ammoniak in das Reaktionsgemisch geleitet. Man rührt weitere 28 Stunden bei 50 °C und leitet dabei ständig einen langsamen Ammoniakstrom durch die Apparatur. Anschließend wird aufgearbeitet, indem man den Biokatalysator abfiltriert, mit tert.-Butanol und Ethanol nachwäscht, das Filtrat unter vermindertem Druck einengt und den verbleibenden Rückstand mit einem Gemisch aus Petrolether:Di-isopropyl-ether = 9:1 verrührt. Das dabei kristallin anfallende Produkt wird abfiltriert, mit Petrolether nachgewaschen und getrocknet. Man erhält auf diese Weise 1,78 g (42,8 % der Theorie, Umsatz 44-Flächenprozent GC) an (R)-2-(4-Chlor-phenyl)-propionsäure-amid mit einem ee-Wert 96,1 %

### Variante β:

Ein Gemisch aus 5 g racemischem 2-(4-Chlor-phenyl)-propionsäure-ethylester und 50 ml tert.-Butanol wird bei Raumtemperatur in einen Autoklaven gegeben und mit 1 g Lipase aus Candida antarctica (Novozym 435, Aktivität 7000 PLU/g) versetzt. Danach kühlt man auf -50 °C und kondensiert 0,5 g Ammoniak in das Reaktionsgemisch. Man verschließt das Druckgefäß, erwärmt auf 50°C und läßt 24 Stunden unter Rühren reagieren. Anschließend wird in der oben beschriebenen Weise aufgearbeitet. Man erhält dabei 1,78 g (42,8 % der Theorie) an (R)-2-(4-Chlor-phenyl)-propionsäureamid mit einem ee-Wert 99,1 %

### 2. Stufe

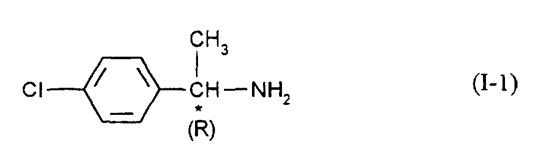

In eine Lösung von 5,5 g (0,137 Mol) Natriumhydroxid in 45 ml Wasser werden bei 0°C unter Rühren 4,4 g (0,0275 Mol) Brom eingetropft. Das entstehende Gemisch wird auf -5°C gekühlt und unter Rühren mit .4,2 g (0,0229 Mol) (R)-2-(4-Chlor-phenyl)-propionsäureamid (ee-Wert 98 %) versetzt. Anschließend wird 5 Stunden auf 50°C erhitzt. Nach dem Abkühlen auf Raumtemperatur extrahiert man das Reaktionsgemisch dreimal mit Methylenchlorid, trocknet die vereinigten organischen Phasen über Natriumsulfat und engt unter vermindertem Druck ein. Auf diese Weise erhält man ein Produkt, das gemäß Gaschromatogramm zu 87 % aus (R)-1-(4-Chlor-phenyl)-ethylamin (ee-Wert >98 %) besteht. Danach errechnet sich eine Ausbeute von 78,5 % der Theorie.

### Beispiel 2

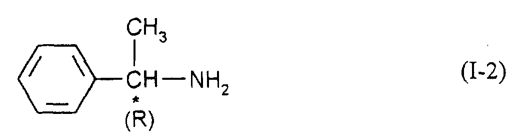

### 1. Stufe:

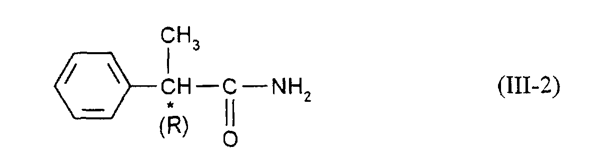

Ein Gemisch aus 1,6 g racemischem 2-Phenyl-propionsäure-ethylester und 18 ml tert.-Butanol wird bei 50°C unter Rühren mit 0,35 g Lipase aus Candida antarctica (Novozym 435, Aktivität 7000 PLU/g) versetzt. Danach wird unter Rühren bei 50 bis 53°C bis zur Sättigung Ammoniak in das Reaktionsgemisch eingeleitet. Man rührt weitere 8 Stunden bei 50 bis 53°C und leitet dabei ständig einen langsamen Ammoniakstrom durch die Apparatur. Anschließend wird in der im Beispiel I angegebenen Weise aufgearbeitet. Man erhält dabei 0,6 g (90 % der Theorie, Umsatz 46 Flächenprozent GC) an (R)-2-Phenyl-propionsäure-amid mit einem ee-Wert >98 %

### 2. Stufe:

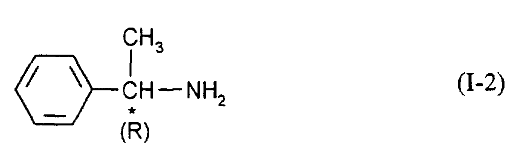

Ein Gemisch aus 6 g (0,04 Mol) (R)-2-Phenyl-propionsäureamid, 4 g (0,1 Mol) Natriumhydroxid und 30 ml Wasser wird bei Raumtemperatur unter Rühren mit 48 g (0,084 Mol) Chlorlauge (Gehalt an aktivem Chlor: 12,5 %) versetzt. Anschließend wird 4 Stunden lang auf 80°C erhitzt. Nach dem Abkühlen auf Raumtemperatur extrahiert man das Reaktionsgemisch dreimal mit je 75 ml Methylenchlorid, trocknet die vereinigten organischen Phasen über Natriumsulfat und engt unter vermindertem Druck ein. Auf diese Weise erhält man 4,7 g eines Produktes, das gemäß Gaschromatogramm zu 85 % aus (R)-1-Phenyl-ethylamin (ee-Wert >98 %) besteht. Danach errechnet sich eine Ausbeute von 82,5 % der Theorie.

## Patentansprüche

1. Verfahren zur Herstellung von (R)-1-Aryl-alkylaminen der Formel 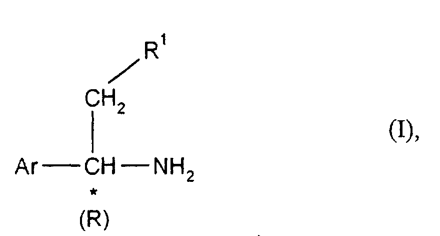 in welcher
R¹ für Wasserstoff oder Alkyl steht und
Ar für gegebenenfalls substituiertes Phenyl der Formel 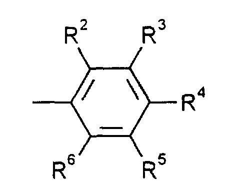 steht, worin
R² und R⁶ ' für Wasserstoff oder Fluor stehen und
R³, R⁴ und R⁵ unabhängig voneinander für Wasserstoff, Halogen, Alkyl, Halogenalkyl, Alkoxy, Halogenalkoxy, Cyano, Dialkylamino, Nitro, Phenyl, Phenoxy oder Benzyl stehen,
oder
Ar für gegebenenfalls einfach bis dreifach, gleichartig oder verschieden durch Halogen, Alkyl, Halogenalkyl, Alkoxy und/oder Halogenalkoxy substituiertes Naphthyl steht,
**dadurch gekennzeichnet, daß** man
a) Racemate von 2-Aryl-alkansäure-ethylestern bzw. - methylestern der Formel 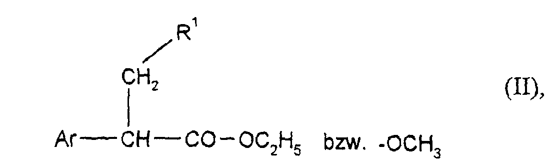 in welcher
R¹ und Ar die oben angegebenen Bedeutungen haben,
mit Ammoniak in Gegenwart von Lipasen, die zur Spaltung von Estern geeignet sind, in Gegenwart eines Verdünnungsmittels umsetzt,
b) aus dem Reaktionsgemisch die entstandenen (R)-2-Aryl-alkansäureamide der Formel 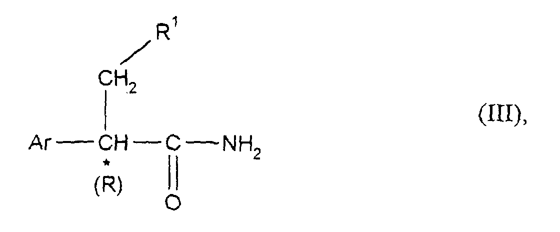
in welcher
R¹ und Ar die oben angegebenen Bedeutungen haben,
abtrennt und dann mit Natrium-hypohalogeniten der Formel
NaOHal (IV),
in welcher
Hal für Chlor oder Brom steht,
in Gegenwart von Wasser umsetzt.

2. Verfahren gemäß Anspruch 1, **dadurch gekennzeichnet, daß** man als Ausgangsstoffe Racemate von 2-Aryl-alkansäure-ethylestern bzw. -methylestern der Formel (II) einsetzt, in denen
R¹ für Wasserstoff oder geradkettiges oder verzweigtes Alkyl mit 1 bis 4 Kohlenstoffatomen steht, und
Ar für gegebenenfalls substituiertes Phenyl der Formel 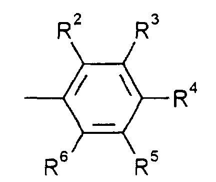 steht, worin
R² und R⁶ für Wasserstoff oder Fluor stehen, wobei zumindest einer dieser beiden Substituenten für Wassersstoff steht, und
R³, R⁴ und R⁵ unabhängig voneinander für Wasserstoff, Fluor, Chlor, Brom, geradkettiges oder verzweigtes Alkyl mit 1 bis 4 Kohlenstoffatomen, geradkettiges oder verzweigtes Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Cyano, Dialkylamino mit 1 bis 4 Kohlenstoffatomen in jeder Alkylgruppe, Nitro, Phenyl, Phenoxy oder Benzyl stehen,
oder
Ar für gegebenenfalls einfach bis dreifach, gleichartig oder verschieden durch Fluor, Chlor, Brom, geradkettiges oder verzweigtes Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen und/oder Halogenalkoxy mit 1 bis 4 Kohlenstoffatomen und 1 bis 5 gleichen oder verschiedenen Halogenatomen substituiertes Naphthyl steht, wobei jedoch die ortho-Positionen zu dem Kohlenstoffatom, über das der Naphthyl-Rest gebunden ist, nicht substituiert sind.

3. Verfahren gemäß Anspruch 1, **dadurch gekennzeichnet, daß** man als Ausgangsstoffe Racemate von 2-Aryl-alkansäure-ethylestern bzw. -methylestern der Formel (II) einsetzt, in denen
R¹ für Wasserstoff, Methyl, Ethyl, n-Propyl, Isopropyl, n-Butyl, iso-Butyl oder sek.-Butyl steht und
Ar für gegebenenfalls substituiertes Phenyl der Formel 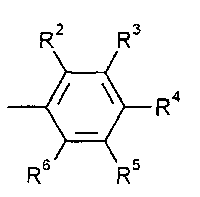 steht, worin
R² und R⁶ für Wasserstoff oder Fluor stehen, wobei zumindest einer dieser beiden Substituenten für Wassersstoff steht, und
R³, R⁴ und R⁵ unabhängig voneinander für Wasserstoff, Fluor, Chlor, Brom, Methyl, Ethyl, n-Propyl, Isopropyl, n-Butyl, iso-Butyl, sek.-Butyl, tert-Butyl, Methoxy, Ethoxy, Trichlormethyl, Trifluormethyl, Difluormethyl, Trifluormethoxy, Difluorchlormethoxy, Difluormethoxy, Cyano, Dimethylamino, Diethylamino, Nitro, Phenyl, Phenoxy oder Benzyl stehen, oder
Ar für gegebenenfalls einfach bis dreifach, gleichartig oder verschieden durch Fluor, Chlor, Brom, Methyl, Ethyl, n-Propyl, Isopropyl, n-Butyl, iso-Butyl, sek.-Butyl, tert.-Butyl, Methoxy, Ethoxy, Trichlormethyl, Trifluormethyl, Difluormethyl, Trifluormethoxy und/oder Difluormethoxy substituiertes Naphthyl steht, wobei jedoch die ortho-Positionen zu dem Kohlenstoffatom, über das der Naphthyl-Rest gebunden ist, nicht substituiert sind.

4. Verfahren gemäß Anspruch 1, **dadurch gekennzeichnet, daß** man Lipase aus Candida antarctica als Biokatalysator einsetzt.

5. Verfahren gemäß Anspruch 1, **dadurch gekennzeichnet, daß** man tertiäre Alkohole, Ether, Acetonitril oder Isopropanol als Verdünnungsmittel bei der Durchführung der ersten Stufe einsetzt.

6. Verfahren gemäß Anspruch 1, **dadurch gekennzeichnet, daß** man bei der Durchführung der ersten Stufe bei Temperaturen zwischen -10°C und 80°C arbeitet.

7. Verfahren gemäß Anspruch 1, **dadurch gekennzeichnet, daß** man bei der Durchführung der zweiten Stufe bei Temperaturen zwischen 0°C und 120°C arbeitet.

## Claims

1. Process for the preparation of (R)-1-arylalkylamines of the formula 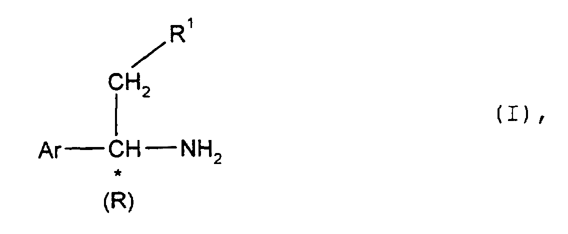 in which
R¹ represents hydrogen or alkyl and
Ar represents optionally substituted phenyl of the formula 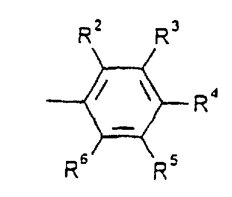 in which
R² and R⁶ represent hydrogen or fluorine and
R³, R⁴ and R⁵ independently of one another represent hydrogen, halogen, alkyl, halogenoalkyl, alkoxy, halogenoalkoxy, cyano, dialkylamino, nitro, phenyl, phenoxy or benzyl,
or
Ar represents naphthyl which is optionally mono- to trisubstituted by identical or different halogen, alkyl, halogenoalkyl, alkoxy and/or halogenoalkoxy substituents,
**characterized in that**
a) racemates of ethyl or methyl 2-aryl-alkanoates of the formula 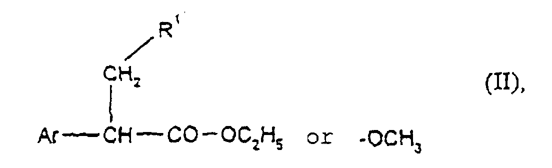 in which
R¹ and Ar have the meanings indicated above,
are reacted with ammonia in the presence of lipases which are suitable for the cleavage of esters, in the presence of a diluent,
b) the resulting (R)-2-aryl-alkanamides of the formula 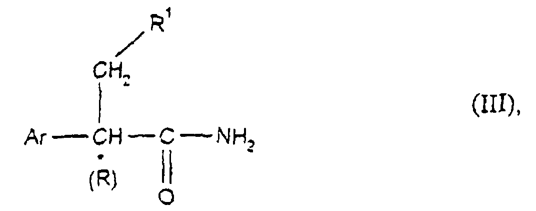 in which
R¹ and Ar have the meanings indicated above,
are separated off from the reaction mixture and then reacted with sodium hypohalites of the formula
NaOHal (IV),
in which
Hal represents chlorine or bromine,
in the presence of water.

2. Process according to Claim 1, **characterized in that**, as starting substances, racemates of ethyl or methyl 2-aryl-alkanoates of the formula (II) are employed, in which
R¹ represents hydrogen or straight-chain or branched alkyl having 1 to 4 carbon atoms, and
Ar represents optionally substituted phenyl of the formula 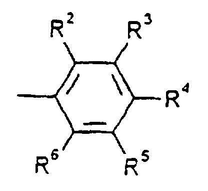 in which
R² and R⁶ represent hydrogen or fluorine, at least one of these two substituents representing hydrogen, and
R³, R⁴ and R⁵ independently of one another represent hydrogen, fluorine, chlorine, bromine, straight-chain or branched alkyl having 1 to 4 carbon atoms, straight-chain or branched alkoxy having 1 to 4 carbon atoms, halogenoalkyl having 1 to 4 carbon atoms and 1 to 5 identical or different halogen atoms, halogenoalkoxy having 1 to 4 carbon atoms and 1 to 5 identical or different halogen atoms, cyano, dialkylamino having 1 to 4 carbon atoms in each alkyl group, nitro, phenyl, phenoxy or benzyl,
or
Ar represents naphthyl which is optionally mono- to trisubstituted by identical or different fluorine, chlorine, bromine, straight-chain or branched alkyl having 1 to 4 carbon atoms, halogenoalkyl having 1 to 4 carbon atoms and 1 to 5 identical or different halogen atoms, alkoxy having 1 to 4 carbon atoms and/or halogenoalkoxy having 1 to 4 carbon atoms and 1 to 5 identical or different halogen atoms, but where the ortho positions to the carbon atom via which the naphthyl radical is bonded are not substituted.

3. Process according to Claim 1, **characterized in that**, as starting substances, racemates of ethyl or methyl 2-aryl-alkanoates of the formula (II) are employed, in which
R¹ represents hydrogen, methyl, ethyl, n-propyl, isopropyl, n-butyl, iso-butyl, sec-butyl or tert-butyl and
Ar represents optionally substituted phenyl of the formula 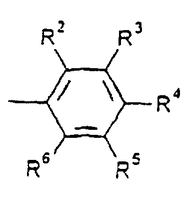 in which
R² and R⁶ represent hydrogen or fluorine, at least one of these two substituents representing hydrogen, and
R³, R⁴ and R⁵ independently of one another represent hydrogen, fluorine, chlorine, bromine, methyl, ethyl, n-propyl, isopropyl, n-butyl, iso-butyl, sec-butyl, tert-butyl, methoxy, ethoxy, trichloromethyl, trifluoromethyl, difluoromethyl, trifluoromethoxy, difluorochloromethoxy, difluoromethoxy, cyano, dimethylamino, diethylamino, nitro, phenyl, phenoxy or benzyl, or
Ar represents naphthyl which is optionally mono- to trisubstituted by identical or different fluorine, chlorine, bromine, methyl, ethyl, n-propyl, isopropyl, n-butyl, iso-butyl, sec-butyl, tert-butyl, methoxy, ethoxy, trichloromethyl, trifluoromethyl, difluoromethyl, trifluoromethoxy and/or difluoromethoxy, but where the ortho positions to the carbon atoms via which the naphthyl radical is bonded are not substituted.

4. Process according to Claim 1, **characterized in that** lipase from Candida antarctica is employed as biocatalyst.

5. Process according to Claim 1, **characterized in that** tertiary alcohols, ether, acetonitrile or isopropanol are employed as diluents in carrying out the first stage.

6. Process according to Claim 1, **characterized in that** the first stage is carried out at temperatures between -10°C and 80°C.

7. Process according to Claim 1, **characterized in that** the second stage is carried out at temperatures between 0°C and 120°C.

## Revendications

1. Procédé de production de (R)-1-arylalkylamines de formule 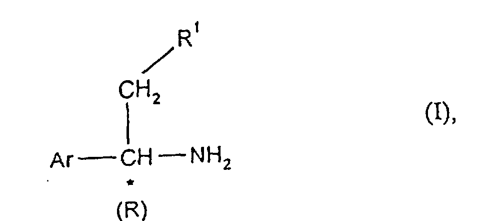 dans laquelle
R¹ représente l'hydrogène ou un groupe alkyle et
Ar représente un groupe phényle éventuellement substitué, de formule 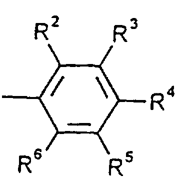 dans laquelle
R² et R⁶ représentent l'hydrogène ou le fluor et
R³, R⁴ et R⁵ représentent, indépendamment les uns des autres, l'hydrogène, un halogène, un groupe alkyle, halogénalkyle, alkoxy, halogénalkoxy, cyano, dialkylamino, nitro, phényle, phénoxy ou benzyle,
ou bien
Ar représente un groupe naphtyle portant éventuellement un à trois substituants, identiques ou différents, halogéno, alkyle, halogénalkyle, alkoxy et/ou halogénalkoxy,
**caractérisé en ce que**
a) on fait réagir des racémates d'esters éthyliques ou d'esters méthyliques d'acides 2-aryl-alcanoïques de formule 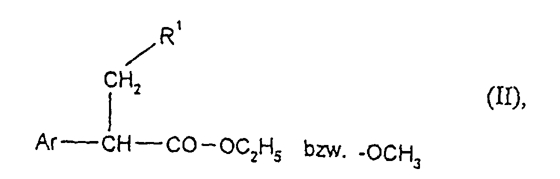 dans laquelle
R¹ et Ar ont les définitions indiquées ci-dessus, avec l'ammoniac en présence de lipases qui conviennent pour scinder des esters, en présence d'un diluant,
b) on sépare du mélange réactionnel les amides d'acides (R)-2-aryl-alcanoïques formés, de formule 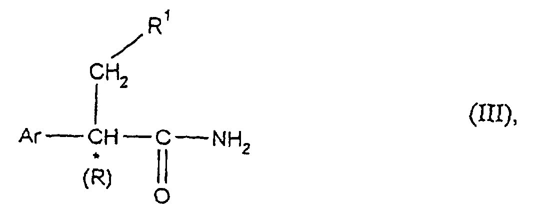 dans laquelle
R¹ et Ar ont les définitions indiquées ci-dessus, puis on les fait réagir avec des hypohalogénites de sodium de formule
NaOHal (IV)
dans laquelle
Hal représente le chlore ou le brome, en présence d'eau.

2. Procédé suivant la revendication 1, **caractérisé en ce qu'**on utilise comme composés de départ des racémates d'esters éthyliques ou d'esters méthyliques d'acides 2-aryl-alcanoïques de formule (II), dans lesquels
R¹ représente l'hydrogène ou un groupe alkyle linéaire ou ramifié ayant 1 à 4 atomes de carbone, et
Ar représente un groupe phényle éventuellement substitué de formule 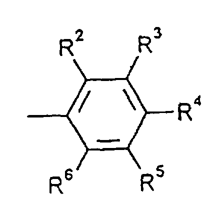 dans laquelle
R² et R⁶ représentent l'hydrogène ou le fluor, l'un au moins de ces deux substituants représentant l'hydrogène, et
R³, R⁴ et R⁵ représentent, indépendamment les uns des autres, l'hydrogène, le fluor, le chlore, le brome, un groupe alkyle linéaire ou ramifié ayant 1 à 4 atomes de carbone, un groupe alkoxy linéaire ou ramifié ayant 1 à 4 atomes de carbone, un groupe halogénalkyle ayant 1 à 4 atomes de carbone et 1 à 5 atomes d'halogènes identiques ou différents, un groupe halogénalkoxy ayant 1 à 4 atomes de carbone et 1 à 5 atomes d'halogènes identiques ou différents, un groupe cyano, dialkylamino ayant 1 à 4 atomes de carbone dans chaque groupe alkyle, un groupe nitro, phényle, phénoxy ou benzyle,
ou bien
Ar représente un groupe naphtyle portant éventuellement un à trois substituants, identiques ou différents, fluoro, chloro, bromo, alkyle linéaire ou ramifié ayant 1 à 4 atomes de carbone, halogénalkyle ayant 1 à 4 atomes de carbone et 1 à 5 atomes d'halogènes identiques ou différents, alkoxy ayant 1 à 4 atomes de carbone et/ou halogénalkoxy ayant 1 à 4 atomes de carbone et 1 à 5 atomes d'halogènes identiques ou différents, les positions ortho par rapport à l'atome de carbone par l'intermédiaire duquel le reste naphtyle est lié n'étant toutefois pas substituées.

3. Procédé suivant la revendication 1, **caractérisé en ce qu'**on utilise comme composés de départ des racémates d'esters éthyliques ou d'esters méthyliques d'acides 2-aryl-alcanoïques de formule (II), dans lesquels
R¹ représente l'hydrogène, un groupe méthyle, éthyle, n-propyle, isopropyle, n-butyle, isobutyle ou sec.-butyle et
Ar représente un groupe phényle éventuellement substitué de formule 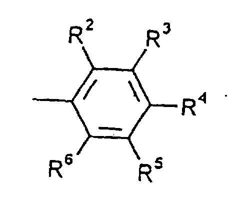 dans laquelle
R² et R⁶ représentent l'hydrogène ou le fluor, au moins l'un de ces deux substituants représentant l'hydrogène, et
R³, R⁴ et R⁵ représentent, indépendamment les uns des autres, l'hydrogène, le fluor, le chlore, le brome, un groupe méthyle, éthyle, n-propyle, isopropyle, n-butyle, isobutyle, sec.-butyle, tertio-butyle, méthoxy, éthoxy, trichlorométhyle, trifluorométhyle, difluorométhyle, trifluorométhoxy, difluorochlorométhoxy, difluorométhoxy, cyano, diméthylamino, diéthylamino, nitro, phényle, phénoxy ou benzyle, ou bien
Ar représente un groupe naphtyle portant éventuellement un à trois substituants, identiques ou différents, fluoro, chloro, bromo, méthyle, éthyle, n-propyle, isopropyle, n-butyle, isobutyle, sec.-butyle, tertiobutyle, méthoxy, éthoxy, trichlorométhyle, trifluorométhyle, difluorométhyle, trifluorométhoxy et/ou difluorométhoxy, les positions ortho par rapport à l'atome de carbone par l'intermédiaire duquel le reste naphtyle est lié n'étant toutefois pas substituées.

4. Procédé suivant la revendication 1, **caractérisé en ce qu'**on utilise comme biocatalyseur la lipase de Candida antarctica.

5. Procédé suivant la revendication 1, **caractérisé en ce qu'**on utilise des alcools tertiaires, des éthers, l'acétonitrile ou l'isopropanol comme diluants dans la conduite de la première étape.

6. Procédé suivant la revendication 1, **caractérisé en ce qu'**on opère à des températures comprises entre -10°C et 80°C dans la conduite de la première étape.

7. Procédé suivant la revendication 1, **caractérisé en ce qu'**on opère à des températures comprises entre 0°C et 120°C dans la conduite de la seconde étape.
